# EUROPÄISCHE PATENTANMELDUNG

(11) **EP 0 724 869 A1**
(43) Veröffentlichungstag der Anmeldung: **07.08.1996**
(21) Anmeldenummer: 96101014.7
(22) Anmeldetag: 25.01.1996
(51) Int. Cl.: A61F 13/08, D04B 21/20, D04B 9/52

(54) **Thrombosestrumpf oder Strumpfteil einer Thrombosestrumpfhose und Verfahren zu ihrer Herstellung**

(30) Priorität: 03.02.1995 DE 19503459
(71) Anmelder: Müller, Stefan, 51674 Wiehl (DE)
(72) Erfinder: Müller, Stefan, 51674 Wiehl (DE)
(74) Vertreter: Neumann, Ernst Dieter, Dipl.-Ing.

(57) **Zusammenfassung**

Thrombosestrumpf (1) oder Strumpfteil einer Thrombosestrumpfhose zur Prophylaxe oder Therapie mit einem anatomisch angepaßten Verlauf mit über der Länge sich verändernder Weite, bei dem zwei Lagen (4,5) doppelkonturig gewirkter Raschelware mit in Längsrichtund des Strumpfes oder Strumpfteils (1) verlaufenden Fadenketten längs ihrer jeweiligen beiden Kanten (6,7) ineinandergewirkt sind, um einen geschlossenen Querschnitt zu bilden und daß die Fadenketten (24,25) der beiden Lagen (4,5) in zumindest einem längs der jeweiligen einen Kanten (6,7) verlaufenden Bereich (8,9) durch in Längsrichtung des Strumpfes oder Strumfteils verlaufende Verbindungsfäden (26) miteinander vermascht sind und daß die Verbindungsfäden (26) gegenüber der Belastung beim Aufweiten des Strumpfes oder Strumpfteils eine geringere Festigkeit als die Garne der Fadenketten (24,25) der beiden Lagen (4,5) haben.

## Beschreibung

Die Erfindung betrifft einen Thrombosestrumpf oder einen Strumpfteil einer Thrombosestrumpfhose zur Prophylaxe oder Therapie mit einem anatomisch angepaßten Verlauf mit über der Länge sich verändernder Weite, bei dem zwei Lagen doppelfonturig gewirkter Raschelware mit in Längsrichtung des Strumpfes oder Strumpfteils verlaufenden Fadenketten längs ihrer jeweiligen beiden Kanten ineinandergewirkt sind, um einen geschlossenen Querschnitt zu bilden.

Aus der EP 0 194 480 B1 sind Strumpfteile der genannten Art als Teile von Strumpfhosen bekannt, die aus zwei Lagen doppelfonturig gewirkter Raschelware bestehen und modischen Zwecken dienen.

Strümpfe dieser Art für medizinische Zwecke sind aus der EP 0 360 731 A2 bekannt und dienen der Prophylaxe gegen Thrombosen - vor, während und nach Operationen - oder der Therapie bei Venenleiden oder Krampfadern. Diese Strümpfe werden mit über der Länge vom Fußteil zum Beinteil zunehmender Weite rundgestrickt, indem die Maschengröße über der Länge vergrößert wird. Dabei ist es bereits bekannt, zur Fixierung des Fußteils die Ferse und den Fußrist bogenförmig auszuformen. Die Strümpfe sollen dabei am Bein des Patienten eine bestimmte äußere Spannung erzeugen, damit der Rückfluß des Blutes, der von den Gefäßen alleine nicht sichergestellt werden kann, gefördert wird. Hierbei ist andererseits die Gefahr des Blutstaus gegenwärtig, so daß die Strümpfe nicht mit zu hoher Spannung getragen werden können bzw. im Verhältnis zur Stärke des Beinumfangs des Patienten beliebig eng angepaßt werden können.

Wünschenswert ist es, eine der Größe nach bestimmte äußere Spannung über der gesamten Länge des Strumpfes bzw. des Beines gleichmäßig abnehmend herzustellen und aufrechtzuerhalten. Dies ist bei den bekannten Strümpfen nicht gegeben. Selbst ein Vorhalten verschiedener Strumpfgrößen kann dieses aufgrund anatomischer Individualität der Patienten nicht sicherstellen. Beispielsweise ist bei einem relativ besonders dick ausgebildeten Kniebereich oder Wadenbereich oder Oberschenkel des Patienten eine Spannungsspitze infolge des dort dann relativ zu engen Strumpfes, d.h. eine übermäßige Wirkung mit der Gefahr eines Blutstaus zu befürchten.

Hiervon ausgehend liegt der Erfindung die Aufgabe zugrunde, einen Strumpf oder ein Strumpfteil der eingangs genannten Art dahingehend zu verbessern, daß er kostengünstig herstellbar ist und daß er in verbesserter Weise an die Anatomie des Beines angepaßt ist.

Die Lösung hierfür besteht darin, daß die Fadenketten der beiden Lagen in zumindest einem längs der einen jeweiligen Kanten verlaufenden Bereich durch in Längsrichtung des Strumpfes oder Strumpfteiles verlaufende Verbindungsfäden miteinander vermascht sind und daß die Verbindungsfäden gegenüber der Belastung beim Aufweiten des Strumpfes oder Strumpfteiles eine geringere Festigkeit als die Garne der Fadenketten der beiden Lagen haben; das heißt, daß die Verbindungsfäden im Verhältnis zu den Garnen der Fadenketten hinsichtlich ihrer Reißfestigkeit derart gestaltet sind, daß sie zunächst elastisch nachgeben und bei Überschreitung des jeweils höchstzulässigen Kompressionsdruckes zerreißen, während die Garne der Fadenketten der beiden Lagen dieser Belastung noch standhalten. Hierbei kann durch das Zerreißen eines Teils der Verbindungsfäden und/oder das Zerreißen von Verbindungsfäden in Teilbereichen ihrer Länge eine richtige und geeignete Spannung über der Länge des Strumpfes oder Strumpfteils im Moment des Anpassens, d. h. des erstmaligen Anziehens selbsttätig hergestellt werden. Damit ist das Auftreten unzulässig hoher Spannungen an irgendeiner Stelle über der Länge des Strumpfes oder Strumpfteiles sicher zu vermeiden. Der hiermit in Rascheltechnik und damit billig auf doppelbarrigen Raschelmaschinen herstellbare Strumpf bzw. das Strumpfteil weist die Möglichkeit auf, jegliche ungleichförmige Änderung der Weite über der Länge vom Fußteil zum Beinteil in Anpassung an die Anatomie so darzustellen, daß längs des gesamten Beines eine angemessene Spannung entsteht.

Zur Anpassung an die durchschnittliche Anatomie wird dabei der Strumpf bzw. das Strumpfteil so hergestellt, daß insgesamt die Maschengröße der beiden Lagen über der Länge vom Fußteil zum Beinteil im wesentlichen zunimmt und/oder die Maschenstäbchendichte der beiden Lagen in der genannten Richtung im wesentlichen abnimmt. Diese Effekte werden durch zunehmende Warenabzugsgeschwindigkeit und abnehmende Fadenspannung bei der Verarbeitung in diesem Richtungssinn und sinngemäß umgekehrt bei Verarbeitung im entgegengesetzten Richtungssinn bewirkt. Im Falle eines Patienten mit der genannten durchschnittlichen Anatomie kommt es zum Aufbau der gewünschten Spannung, ohne daß die Verbindungsfäden beschädigt werden. Es ist vorgesehen, daß jeweils mehrere parallel zueinander verlaufende Verbindungsfäden die Bereiche bilden, in denen die beiden Lagen miteinander vermascht sind.

Infolge der Verbindung der beiden Lagen durch die Verbindungsfäden entstehen außerhalb der effektiven offenen Strumpfweite durch die Verbindungsfäden erzeugte Fahnen, die für die Funktion des Strumpfes oder Strumpfteils keine unmittelbare Bedeutung haben.

Nach einer ersten Ausgestaltung ist es möglich, daß nur eine Gruppe von Verbindungsfäden die beiden Lagen längs der jeweiligen einen Kanten parallel zu diesen vermaschen, während an den jeweiligen gegenüberliegenden Kanten keine zusätzlichen Verbindungfäden eingesetzt werden. Hierbei ist zwar nur eine Gruppe von Verbindungsfäden zu verwenden, die einseitige Fahne kann aber ggfs. unerwünscht breit werden. In einer zweiten Form kann vorgesehen sein, daß zwei Gruppen von Verbindungsfäden die beiden Lagen längs der jeweiligen beiden Kanten mit Abstand zu diesen vermaschen. Hierbei sind zwar zwei Gruppen von Verbindungsfäden zu verwenden, jedoch bilden sich beidseitig zur effektiven offenen Weite jeweils nur kleinere Fahnen aus.

Mit dem Begriff Gruppe von Verbindungsfäden ist ggfs. auch das Minimum eines einzigen Verbindungsfadens gemeint, in der Regel werden jedoch Gruppen von zwei oder mehr parallelen Verbindungsfäden verwendet werden.

Die erfindungsgemäß auf Raschelmaschinen hergestellten Strümpfe oder Strumpfteile sind so kostengünstig zu erzeugen, daß in der Regel nur eine einmalige Verwendung erfolgt.

Die beiden Lagen können zur Erzeugung eines geschlossenen Fußteils quer zur Längsrichtung des Strumpfes oder Strumpfteils ineinandergewirkt werden. Zur Erzeugung eines Fensters für die Zehen am Fußteil kann in einer der Lagen ein in Längsrichtung des Strumpfes oder Strumpfteils verlaufender Schlitz vorgesehen sein. Hiermit ist eine regelmäßige Kontrolle einer ausreichenden Blutzirkulation bis zum Fuß möglich.

Ein geeignetes Verfahren zur Herstellung von Strümpfen der erfindungsgemäßen Art besteht darin, daß ein endloser Schlauch hergestellt wird, der Übergangs- und Verschnittbereiche jeweils zwischen zwei aufeinanderfolgenden Strümpfen aufweist. Die Bereiche zwischen zwei Strümpfen werden in einem nachgeschalteten Verfahrensschritt durchtrennt oder herausgetrennt, wobei die vorzugsweise als offene Fransen gelegten Maschen gesichert sein müssen.

Dies geschieht durch Annähen eines Bündchens, das gleichzeitig als Rutschsicherung beim Tragen dient und z.B. aus gummiertem Material bestehen kann. Um sprungartige Übergänge vom Fußteil eines Strumpfes zum Beinteil eines anderen Strumpfes zu vermeiden, die einen großen Übergangs- und Verschnittbereich bedingen würden, ist vorzugsweise vorgesehen, daß ein endloser Schlauch hergestellt wird, an dem wechselweise zwei Fußteile und zwei Beinteile jeweils aufeinanderfolgender Strümpfe unmittelbar aufeinander folgen. Hierbei ist ein reiner Abtrennvorgang ohne Verschnitt für die einzelnen Strümpfe möglich.

Eine weitere Verfahrensart zur Herstellung von Strumpfteilen besteht darin, daß zwei parallel zueinander verlaufende Schläuche zur Bildung zweier Strumpfteile hergestellt werden, die zur Erzeugung des Rumpfteiles einer Strumpfhose abschnittsweise zu einem gemeinsamen Schlauch verbunden werden. Bei Bedarf können so anstelle zweier Strümpfe den betroffenen Patienten Strumpfhosen zur Verfügung gestellt werden, die die gewünschte Spannung über die volle Beinlänge bis in den Rumpfbereich aufrechterhalten. Auch hierbei ist zum Zweck einer rationellen, verschnittfreien Fertigung vorzugsweise vorgesehen, daß endlose Schläuche hergestellt werden, an denen wechselweise die Fußteile und die Rumpfteile jeweils aufeinanderfolgender Strumpfhosen unmittelbar aufeinander folgen. Auch hierbei sind die durch anschließendes Abtrennen entstehenden Strumpfhosen durch Annähen eines Bündchens aus rutschfestem Material an der Rumpföffnung fertig zu konfektionieren.

Soweit nur einfache Strümpfe verwendet werden, kann es sich sowohl um Kniestrümpfe als auch um lange Strümpfe handeln, die entsprechend der Diagnose des behandelnden Arztes zum Einsatz gebracht werden. Abwandlungen und Ausgestaltungen des Vorstehendenden, insbesondere die Wahl der Garne und der Legungsarten sind der Auswahl des Fachmannes überlassen.

Ein bevorzugtes Ausführungsbeispiel wird nachstehend anhand der Zeichnungen beschrieben.
- Figur 1: zeigt einen erfindungsgemäßen Strumpf in einer ersten Ausführung in Ansicht (a) und im Querschnitt (b);
- Figur 2: zeigt einen erfindungsgemäßen Strumpf in einer zweiten Ausführung in Ansicht (a) und im Querschnitt (b);
- Figur 3: zeigt mehrere erfindungsgemäße Strümpfe bei der Herstellung;
- Figur 4: zeigt mehrere erfindungsgemäße Strumpfhosen bei der Herstellung;
- Figur 5: zeigt systematisch zwei Ausschnitte der beiden Lagen eines doppelfonturigen Gewirkes im Bereich ihrer Verbindung durch die Verbindungsfäden.

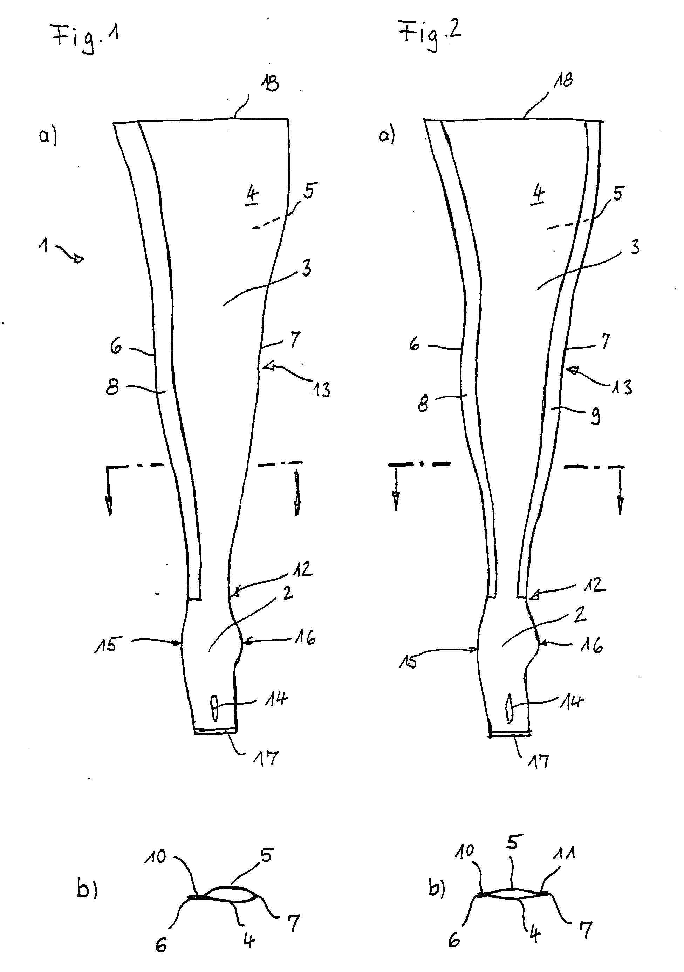

In den Figuren 1 und 2 ist ein schlauchförmiger Strumpf 1 dargestellt, dessen Fadenketten in Längsrichtung des Strumpfes verlaufen. Der Abstand der Maschenstäbchen nimmt vom Fußteil 2 zum Beinteil 3 über der Länge im wesentlichen zu. Das gleiche gilt vorzugsweise für die Maschenweite, ohne daß dies besonders dargestellt ist, wobei jedoch die Anpassung an eine durchschnittliche Anatomie berücksichtigt ist. Es ist erkennbar, daß insbesondere im Fesselbereich 12 und im Kniekehlenbereich 13 des Strumpfes eine Weitenverringerung erfolgt, um dort trotz schwächeren Verlaufs des Beines den erforderlichen Druck aufrechtzuerhalten. Demgegenüber sind für den Fußrist 15 und die Ferse 16 Erweiterungen in der Verarbeitung der Fadenketten ausgebildet. Der gezeigte Strumpf ist grundsätzlich aus zwei Lagen 4 (obere Lage) und 5 (untere Lage) hergestellt, die längs erster Kanten 6 und zweiter Kanten 7 miteinander verbunden sind, d. h. die die Lagen bildenden Fäden sind unter Ausbildung kaum erkennbarer Nahtbereiche ineinandergewirkt. Im Fußbereich 2 ist in der oberen Lage 4 eine Öffnung 14 für die Zehen in Form eines längs verlaufenden Schlitzes vorgesehen. Unterhalb dieser sind die Lagen 4 und 5 in einem Nahtbereich 17 ineinandergewirkt. In Figur 1 verläuft längs der Kanten 6 und parallel dazu ein Bereich 8 von Verbindungsfäden, die die beiden Lagen 4, 5 unmittelbar im Material miteinander vermaschen, wobei durch die Verbindungsfäden eine Fahne 10 entsteht, die üblicherweise außen vom Strumpf getragen wird, um keine Druckstellen am Bein zu erzeugen. In Figur 2 verlaufen längs beider Kanten 6, 7 Bereiche 8, 9 von Verbindungsfäden, wobei die Breite der durch die Verbindungsfäden gebildeten Fahnen 10, 11 etwa jeweils halb so breit wie die der einzelnen Fahne 10 in Figur 1 ist. Jeder Bereich 8, 9 von Verbindungsfäden besteht aus einer Mehrzahl von Einzelfäden, wobei diese leichter zerreißbar sind als das Grundgewirke, so daß ein Aufreißen einzelner Fäden, ggfs. auch über Teillängen eine selbständige Erweiterung und Anpassung des Strumpfes an das Bein bei sonst unzulässig hoher Spannung ermöglichen würden.

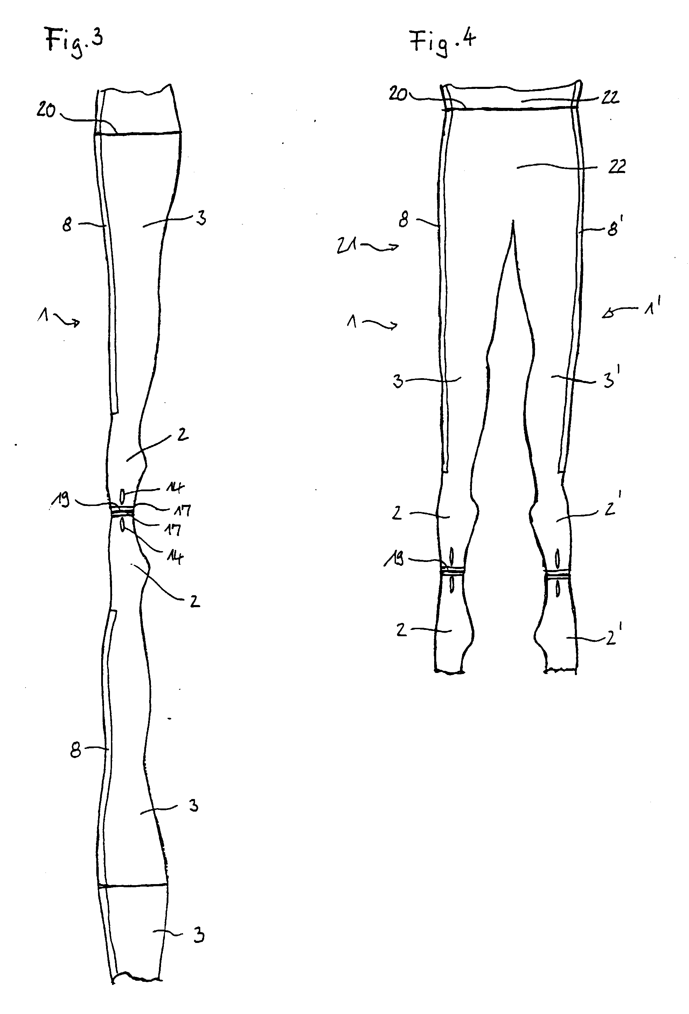

In Figur 3 sind mehrere fortlaufend hergestellte Strümpfe 1 erkennbar, die so angeordnet sind, daß sich jeweils ein Fußteil 2 des einen Strumpfes an das Fußteil 2 des anderen Strumpfes und ein Beinteil 3 des einen Strumpfes an das Beinteil 3 des nächsten Strumpfes anschließt. An Einzelheiten sind die Bereiche 8 mit Verbindungsfäden und die Zehenöffnungen 14 erkennbar, weiterhin die Nahtbereiche 17 an den Fußteilen 2, die mittig nach der Herstellung durchtrennt werden, wie durch eine mittlere Trennlinie 19 angedeutet. Zwischen den einzelnen Beinteilen 3 gehen die Beinöffnungen offen ineinander über; hier werden die Strümpfe nach der Herstellung im offenen Querschnitt durchtrennt, wie durch die Trennlinien 20 dargestellt ist. Die Beinöffnungen werden anschließend mit Bündchen befestigt.

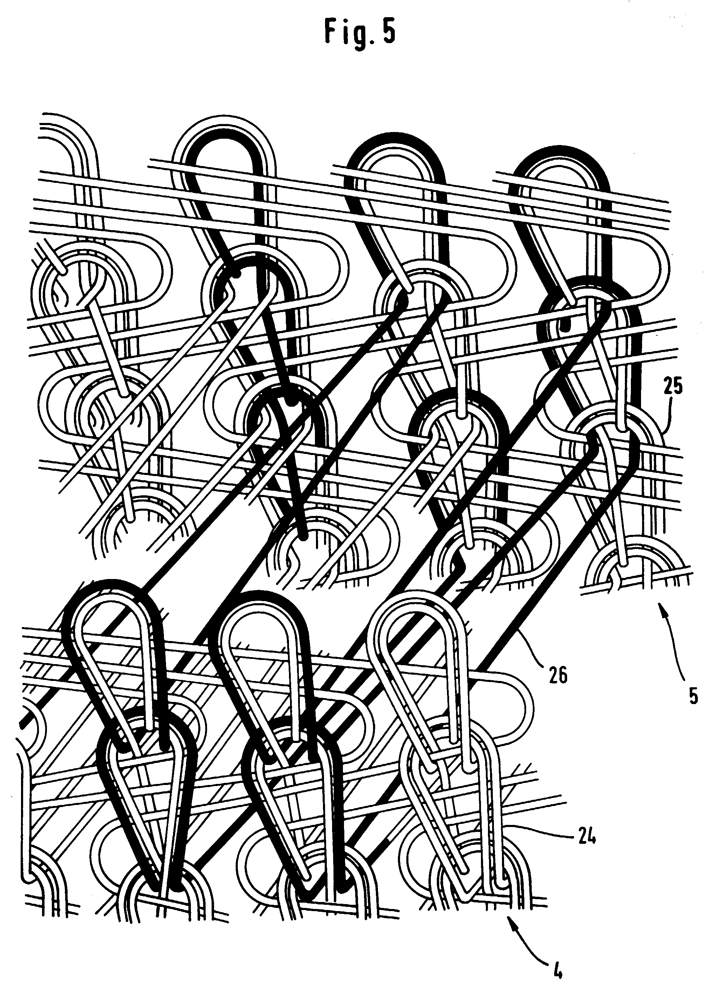

In Figur 4 sind mehrere fortlaufend hergestellte Strumpfhosen 21 erkennbar, die jeweils aus zwei Beinteilen 1, 1' bestehen, die ineinander übergehen und ein Rumpfteil 22 bilden. Auch hier sind Bereiche 8, 8' mit Verbindungsfäden erkennbar, die leichter zerreißbar sind als das Grundmaterial. Die Nahtbereiche 17 sind in gleicher Weise zum Abschluß der Fußteile 2 ausgebildet wie in Figur 3. Mittig zwischen zwei Nahtbereichen verläuft eine Trennlinie 19. Eine Trennlinie 20 ist zwischen den Rumpfbereichen 22 dargestellt. Nach dem Trennen müssen die Rumpföffnungen mit Bündchen befestigt werden.

In allen Figuren sind die Verbindungsbereiche 8, 9 jeweils nur bis oberhalb der Fesselbereiche 12 geführt, es ist jedoch auch denkbar, diese durchlaufend bis ins Ende des Fußteils 2 auszugestalten.

In Figur 5 ist ein Ausschnitt aus einem der Verbindungsbereiche in Vergrößerung gezeigt. Es ist eine obere Lage 4 mit parallelen Fadenketten 24, eine untere Lage 5 mit parallelen Fadenketten 25 und deren Verbindung durch eine Gruppe von mehreren Verbindungsfäden 26 dargestellt. Die Fadenketten 24, 25 sind in Umrissen dargestellt, die Verbindungsfäden 26 mit voll ausgezogenen Linien. Die Legungsart der die Lagen bildenden Fäden und die Einbeziehung der Verbindungsfäden ist anhand dieses Ausführungsbeispiels erkennbar. Abwandlungen der Wirkart sind dem Fachmann möglich.

### Bezugszeichenliste

1 Strumpf
2 Fußteil
3 Beinteil
4 obere Lage
5 untere Lage
6 erste Kanten
7 zweite Kanten
8 Verbindungsfäden, erste
9 Verbindungsfäden, zweite
10 Fahne
11 Fahne
12 Fesselbereich
13 Kniekehlenbereich
14 Zehenöffnung
15 Ristbereich
16 Fersenbereich
17 Nahtbereich
18 Beinöffnung
19 Trennlinie
20 Trennlinie
21 Strumpfhose
22 Rumpfteil
24 Fadenkette (4)
25 Fadenkette (5)
26 Verbindungsfäden

## Patentansprüche

1. Thrombosestrumpf (1) oder Strumpfteil einer Thrombosestrumpfhose zur Prophylaxe oder Therapie mit einem anatomisch angepaßten Verlauf mit über der Länge sich verändernder Weite, bei dem zwei Lagen (4, 5) doppelfonturig gewirkter Raschelware mit in Längsrichtung des Strumpfes oder Strumpfteils (1) verlaufenden Fadenketten längs ihrer jeweiligen beiden Kanten (6, 7) ineinandergewirkt sind, um einen geschlossenen Querschnitt zu bilden,
dadurch gekennzeichnet,
daß die Fadenketten (24, 25) der beiden Lagen (4, 5) in zumindest einem längs der jeweiligen einen Kanten (6, 7) verlaufenden Bereich (8, 9) durch in Längsrichtung des Strumpfes oder Strumpfteils verlaufende Verbindungsfäden (26) miteinander vermascht sind und daß die Verbindungsfäden (26) gegenüber der Belastung beim Aufweiten des Strumpfes oder Strumpfteils eine geringere Festigkeit als die Garne der Fadenketten (24, 25) der beiden Lagen (4, 5) haben.

2. Strumpf oder Strumpfteil nach Anspruch 1,
dadurch gekennzeichnet,
daß die Fadenketten (24, 25) der beiden Lagen (4, 5) in beiden längs der jeweiligen beiden Kanten (6, 7) verlaufen den Bereichen (8, 9) durch Verbindungsfäden (26) miteinander vermascht sind.

3. Strumpf oder Strumpfteil nach einem der Ansprüche 1 oder 2,
dadurch gekennzeichnet,
daß die Fadenketten (24, 25) der beiden Lagen (4, 5) zur Erzeugung eines geschlossenen Fußteils (2) in einem Bereich (17) quer zur Längsrichtung des Strumpfes (1) ineinandergewirkt sind.

4. Strumpf oder Strumpfteil nach Anspruch 3,
dadurch gekennzeichnet,
daß in einer der beiden Lagen (4, 5) zur Erzeugung eines Fensters für die Zehen am Fußteil (2) eine in Längsrichtung des Strumpfes (1) verlaufende Öffnung (14) ausgebildet ist.

5. Verfahren zur Herstellung eines Strumpfes oder Strumpfteils nach einem der Ansprüche 1 bis 4,
dadurch gekennzeichnet,
daß zur Erzeugung einer insgesamt im wesentlichen zunehmenden Maschengröße vom Fußteil (2) zur Beinöffnung (18) die beiden Lagen (4, 5) mit in der genannten Richtung zunehmender Warenabzugsgeschwindigkeit bei der Verarbeitung hergestellt sind.

6. Verfahren zur Herstellung eines Strumpfes oder Strumpfteils nach einem der Ansprüche 1 bis 4,
dadurch gekennzeichnet,
daß zur Erzeugung einer abnehmenden Maschenstäbchendichte vom Fußteil (2) zur Beinöffnung (18) die beiden Lagen (4, 5) mit in der genannten Richtung abnehmender Fadenspannung bei der Verarbeitung hergestellt sind.

7. Verfahren zur Herstellung von Strümpfen nach einem der Ansprüche 1 bis 4,
dadurch gekennzeichnet,
daß aus den beiden Lagen (4, 5) ein endloser Schlauch hergestellt wird, der Übergangs- und Verschnittbereiche (19, 20) jeweils zwischen zwei aufeinanderfolgenden Strümpfen aufweist.

8. Verfahren zur Herstellung von Strümpfen nach Anspruch 7,
dadurch gekennzeichnet,
daß ein endloser Schlauch hergestellt wird, an dem wechselweise zwei Fußteile (2) und zwei Beinteile (3) jeweils aufeinanderfolgender Strümpfe (1) unmittelbar aufeinander folgen.

9. Verfahren zur Herstellung von Strumpfteilen nach einem der Ansprüche 1 bis 4,
dadurch gekennzeichnet,
daß aus den beiden Lagen (4, 5) zwei parallel zueinander verlaufende Schläuche zur Bildung zweier Strumpfteile hergestellt werden, die zur Erzeugung des Rumpfteiles (22) einer Strumpfhose (1) zu einem gemeinsamen Schlauch verbunden werden.

10. Verfahren zur Herstellung von Strumpfteilen nach Anspruch 9,
dadurch gekennzeichnet,
daß endlose Schläuche hergestellt werden, an denen wechselweise zwei Fußteile (2) und zwei Rumpfteile (22) jeweils aufeinanderfolgender Strumpfhosen (21) unmittelbar aufeinander folgen.
